Europäisches Patentamt

European Patent Office

Office européen des brevets

(11) Veröffentlichungsnummer: **0 507 047 A2**

# EUROPÄISCHE PATENTANMELDUNG

(21) Anmeldenummer: **92101210.0**

(22) Anmeldetag: **25.01.92**

(51) Int. Cl.5: **B01F 17/56**, A61K 7/00, A61K 7/075

(30) Priorität: **30.03.91 DE 4110506**

(43) Veröffentlichungstag der Anmeldung:
**07.10.92 Patentblatt 92/41**

(84) Benannte Vertragsstaaten:
**AT BE CH DE DK ES FR GB GR IT LI LU NL SE**

(71) Anmelder: **HÜLS AKTIENGESELLSCHAFT
Patentabteilung / PB 15 - Postfach 13 20
W-4370 Marl 1(DE)**

(72) Erfinder: **Balzer, Dieter, Dr.
Talstrasse 21
W-4358 Haltern(DE)**

(54) **Emulgatoren zur Herstellung von in der Kosmetik oder Medizin verwendbaren Öl-in-Wasser-Emulsionen etherischer Öle.**

(57) 2.1 Aufgrund des speziellen Einsatzgebietes sind Emulgatorsysteme gefragt, die außergewöhnlich hohe Haut- bzw. Schleimhautverträglichkeiten aufweisen.

2.2 Es werden Alkylpolyglycoside der Formel

$$R - O - Z_n$$

als Emulgatoren eventuell in Verbindung geringer Mengen üblicher Coemulgatoren vorgeschlagen.

EP 0 507 047 A2

Die Erfindung betrifft Emulgatoren für die Herstellung von Emulsionen etherischer Öle oder deren Mischungen mit anderen in der Kosmetik oder Medizin verwendeten Ölen oder Fettphasen.

Zubereitungen solcher Art finden mannigfache Verwendung bei Badeölen, Badeemulsionen (Bademilch) sowie kosmetischen Reinigungsflüssigkeiten, die etherische Öle enthalten. Da die etherischen Öle teilweise auch antiseptische Wirkungen besitzen, zielt die Erfindung auch auf medizinische Bade- bzw. Einreibeflüssigkeiten. Die Ölphase dieser Flüssigkeiten kann hierbei entweder emulgiert oder - was häufig durch höhere Emulgatorkonzentrationen gelingt - solubilisiert sein.

Solche Emulsionen werden seit langem vermarktet. Als Emulgatoren dienen hierbei Tenside zum Beispiel Alkoholsulfate, Betaine sowie Oxethylate von Fettalkoholen, Phosphorsäureestern, Sorbitanestern, etc. oder deren Gemische. Aufgrund der speziellen kosmetisch oder medizinischen Anwendungen dieser Öl-in-Wasser-Emulsionen ist eine außergewöhnlich hohe Haut- und Schleimhautverträglichkeit Voraussetzung für die eingesetzten Emulgatoren.

Es bestand daher die Aufgabe, Emulgatoren zu finden, die einen außergewöhnlich hohe Haut- und Schleimhautverträglich bei gleichzeitig hoher Emulgierfähigkeit für die entsprechenden in der Kosmetik oder Medizin üblicherweise verwendeten Öl- oder Fettphasen aufweisen.

Die Aufgabe wurden durch den Einsatz von speziellen Alkylpolyglycosiden als Emulgatoren gelöst.

Gegenstand der Erfindung sind daher Emulgatorsysteme zur Herstellung von in der Kosmetik oder Medizin verwendbaren Öl-in-Wasser-Emulsionen etherischer Öle allein oder im Gemisch mit einer Fettphase, welche dadurch gekennzeichnet sind, daß sie Alkylpolyglycoside der Formel (I)

$$R - O - Z_n \qquad (I)$$

enthalten, wobei R für einen linearen oder verzweigten, gesättigten oder ungesättigten Alkylrest mit 8 - 16 C-Atomen, Z für einen Oligoglycosidrest stehen und n im Mittel 1 - 5 bedeutet.

Es wurde überraschend gefunden, daß diese überaus milden Tenside allein oder zum Teil in Kombination mit geringen Mengen in der Kosmetik oder Medizin üblichen Tensiden hervorragende Emulgatoren für etherische Öle sowie auch deren Kombination mit anderen in der Kosmetik oder Medizin üblichen Fettphasen sind.

Dies ist umso überraschender, da es aus der DE-OS 39 25 846 bekannt war, daß sich Alkylpolyglycoside als Emulgatoren für Polysiloxan- oder Polysiloxan-Paraffinöl-Emulsionen hervorragend eignen, somit also für Öle eignen, die durch viel niedrigere HLB-Werte charakterisiert sind als sie für etherische Öle gelten.

Es wurde weiterhin beobachtet, daß es zur Herstellung dieser lagerstabilen Öl-in-Wasser-Emulsionen keiner hohen Scherkräfte bedarf; in manchen Fällen, besonders in Gegenwart hoher Konzentrationen, entstehen hierbei transparente Systeme (vermutlich Mikroemulsionen).

Die Hautfreundlichkeit für Alkylpolyglycoside ist allgemein bekannt. Wie aus der Arbeit G. Proserpio, G. Vianello, Applicazioni tensio-cosmetiche di un nuovo glucoside, Rivista Italiana, essenze, profumi, piante officinali, aromi, saponi, cosmetici, aerosol, Nr. 10, Oktober 1974, Seiten 567 bis 512 hervorgeht, ist z. B. das Alkylpolyglycosid Triton CG 110 ($C_8$-$C_{10}$-Alkoholbasis) hautverträglicher als viele andere bei kosmetischen Zubereitungen übliche Tenside. Auch F. A. Hughes und B. W. Lew, J.A.O.C.S. 47, 162 (1970) beschreiben die hervorragende Haut- und Schleimhautverträglichkeit der Alkylpolyglycoside.

Alkylpolyglycoside

Als spezielle erfindungsgemäß zu verwendende Alkylpolyglycoside kommen Alkylpolyglycoside in Frage, die der Formel (I)

$$R - O - Z_n \qquad (I)$$

entsprechen, wobei R für einen linearen oder verzweigten, gesättigten oder ungesättigten Alkylrest mit 8 - 16 C-Atomen, Z für einen Oligoglycosidrest stehen und n im Mittel 1 - 5 bedeutet.

Besonders vorzuziehen sind lineare gesättigte Alkylpolyglycoside mit 10 bis 14 Kohlenstoffatomen mit einem Glycosidierungsgrad zwischen 1.1 und 2.

Die erfindungsgemäß eingesetzten Alkylpolyglycoside können nach bekannten Verfahren ganz oder teilweise auf Basis nachwachsender Rohstoffe hergestellt werden. Beispielsweise wird Dextrose in Gegenwart eines sauren Katalysators mit n-Butanol zu Butyloligoglycosidgemischen umgesetzt, welche mit langkettigen Alkoholen ebenfalls in Gegenwart eines sauren Katalysators zu den gewünschten Alkyloligoglycosidgemischen umglycosidiert werden. Die Formel der Produkte ist in bestimmten Grenzen variierbar. Der Alkylrest R wird durch die Auswahl des langkettigen Alkohols festgelegt.

Günstig aus wirtschaftlichen Gründen sind die großechnischen zugänglichen Tensidalkohole mit 8 bis 16 C-Atomen, z. B. native Alkohole aus der Hydrierung von Fettsäuren bzw. Fettsäurederivaten, Ziegleralkohole und eventuell auch Oxoalkohole.

Der Oligoglycosylrest $Z_n$ wird einerseits durch die Auswahl des Kohlenhydrats und andererseits durch die Einstellung des mittleren Oligomerisationsgrades n z. B. nach DE-OS 19 43 689 festgelegt. Im Prinzip können bekanntlich Polysaccharide, Oligosaccharide und Monosaccharide, z. B. Stärke, Maltodextrine, Dextrose, Galaktose, Mannose, Xylose usw. zu Alkylpolyglycosiden umgesetzt werden. Besonders bevorzugt sind die großtechnisch verfügbaren Kohlenhydrate Stärke, Maltodextrine und Dextrose. Da die wirtschaftlich interessanten Alkylpolyglycosidsynthesen nicht regio- und stereoselektiv verlaufen, sind die Alkylpolyglycoside stets Gemische von Oligomeren, die ihrerseits Gemische verschiedener isomerer Formen darstellen. Sie liegen nebeneinander mit $\alpha$- und $\beta$-glycosidischen Bindungen in Pyranose- und Furanoseform vor. Auch die Verknüpfungsstellen zwischen zwei Saccharidresten sind unterschiedlich.

Synthesebedingt können die Alkylpolyglycoside auch Begleitsubstanzen wie Restalkohole, Monosaccharide, Oligosaccharide und Oligoalkylpolyglycoside enthalten.

Erfindungsgemäß eingesetzte Alkylpolyglycoside lassen sich auch durch Abmischen von Alkylpolyglycosiden mit Alkylmonoglycosiden herstellen. Letztere kann man z. B. nach EP-A 0 092 355 mittels polarer Lösemittel, wie Aceton, aus Alkylpolyglycosiden gewinnen bzw. anreichern.

Der Glycosidierungsgrad wird zweckmäßigerweise mittels $^1$H-NMR bestimmt.

Ökologisch zählen die Alkylpolyglycoside zu den umweltfreundlichsten Tensiden. So wurden bei Prüfungen der biologischen Abbaubarkeit (Coupled Unit Test, DOC-Messung) Werte von 95 bis 97 % erreicht.

Die Toxizitätsdaten mit LD 50 (Ratte) > 10.000 mg/kg, LC 50 (Goldorfe) 12 - 40 mg/l und EC 50 (Daphnia) 30 - 110 mg/l bei je 2 $C_{10}$ $C_{12}$- bzw. $C_{12}C_{14}$-Alkylpolyglycosiden deuten ebenfalls eine im Vergleich mit vielen anderen Tensiden hervorragendes Umweltverhalten an.

Ähnliches gilt für die Daten der Haut- und Schleimhautirritation, die für $C_{10}C_{12}$-, $C_{12}C_{14}$- und $C_{12}C_{13}$-Alkylpolyglycoside mit Glycosidierungsgraden zwischen 1.2 und 1.7 im Vergleich zu allen anderen getesteten Tensiden äußerst günstig liegen. Folglich ist diese Tensidgruppe allein oder in Kombination mit geringen Mengen anderer Tenside (Coemulgatoren), wodurch deren Reizpotential herabgesetzt wird, für kosmetische und auch medizinische Anwendungen prädestiniert.

Der erfindungsgemäße Gehalt der Alkylpolyglycoside am Emulgatorsystem beträgt 80 bis 100 %, vorzugsweise 85 bis 100 %.

## Coemulgatoren

Erfindungsgemäße Coemulgatoren sind möglichst hautfreundliche Tenside, die in Kombination mit den Hauptemulgatoren, den Alkylpolyglycosiden, zu lagerstabilen, feinteiligen Emulsionen führen. Sie werden dann eingesetzt, wenn das Alkylpolyglykosid oder Kombinationen verschiedener Alkylpolyglykoside allein nicht zum Ziel führen. Ihre Mengen sind im Vergleich zum Hauptemulgator gewöhnlich klein, so daß ihre meist höhere Unverträglichkeit gegenüber Haut und Umwelt durch das im Überschuß vorhandene Alkylpolyglycosid stark erniedrigt wird.

Erfindungsgemäße Coemulgatoren sind Fettalkoholsulfate, Fettalkoholethersulfate, Alkansulfonate, carboxymethylierte Fettalkoholoxethylate, Fettalkohol(ether)phosphate, Fettalkoholethersulfosuccinate, Alkylbetaine, Fettalkoholoxethylate, Fettsäureoxethylate, Fettsäureester von mehrwertigen Alkoholen bzw. von ethoxylierten mehrwertigen Alkoholen (Sorbitanester), Zuckerfettsäureester, Fettsäureteilglyceride, Glyceride mit Fettsäuren und mehrwertigen Carbonsäuren, etc., wobei die Kettenlänge der gesättigten oder ungesättigten Alkylkette jeweils $C_8$-$C_{20}$, vorzugsweise $C_{10}$-$C_{18}$ beträgt und die Kationen der anionischen Tenside Na, K, $NH_4$, $C_2$-$C_3$-Alkanolammonium oder Mg sind. Die Ethoxylierungsgrade liegen bei den Fettalkoholethersulfaten zwischen 1 bis 5 (vorzugsweise zwischen 2 und 4), bei den carboxylierten Oxethylaten zwischen 2 bis 15 (3 - 10), bei den Fettalkoholethersulfosuccinaten zwischen 1 bis 6 (2 - 4), bei den Fettalkoholetherphosphaten zwischen 0 - 12 (2 - 10), bei den Fettalkoholoxethylaten zwischen 3 bis 30 (4 - 25), bei den Fettsäureoxethylaten zwischen 2 bis 25 (3 - 20), bei den Fettsäureester mehrwertiger ethoxylierter Alkohole zwischen 3 - 30 (vorzugsweise 4 bis 20) mol Ethylenoxid/mol.

Außer diesen Tensiden und ihren Gemischen kommen weitere Produkte in Frage, wie sie zum Beispiel von B. Idson in Surfactants in Cosmetics, Ed. M. M. Rieger, Surfactant Science Series, Vol. 16, S. 1 bis 28 dargestellt werden.

Der Gehalt des Coemulgators am Emulgatorsystem liegt zwischen 0 und 20 %, vorzugsweise zwischen 0 und 15 %.

## Weitere Bestandteile

Weitere Bestandteile der Emulsion sind die zu emulgierende Phase, Wasser, ggf. Elektrolyt, Farbstoffe, Konservierungsmittel, sogenannte Rückfetter wie zum Beispiel ethoxylierte Partialglyceride, etc.

Die zu emulgierende Phase besteht aus etherischen Ölen wie z. B. Lavendel-, Kiefernnadel-, Fichtennadel-, Eukalyptus-, Orangen-, Rosmarin-, Thymian-, Citronenöl, etc. bzw. deren Gemische häufig in Abmischung mit Fettphasen wie Erdnußöl, Ölivenöl, Paraffinöl, Isopropylmyristat, etc.

Eine bevorzugte Verfahrensweise zur Herstellung der Emulsion besteht nun darin, daß der erfindungsgemäße Emulgator bei Raumtemperatur in dem etherischen Öl bzw. Gemisch aus etherischen Öl und Fettphase gelöst oder dispergiert wird und sodann mit geringem Scheraufwand in der wäßrigen Phase verteilt wird. Bei einer anderen bevorzugten Methode wird der Emulgator in einer wäßrigen Phase gelöst oder dispergiert und sodann unter geringem Scheraufwand die Fettphase zugemischt. Die bei beiden Verfahren erzielten Emulsionen sind gewöhnlich von niedriger bis mittlerer Viskosität (1 - 500 mPa•s), bläulich-weißem bis weißem Aussehen, sofern die Ölphase ungefärbt ist, und selbst bei erhöhter Temperatur (bis ca. 50 °C) völlig lagerstabil. In manchen Fällen besonders in Gegenwart erhöhter Emulgatorkonzentrationen entstehen transparente Phasen, vermutlich Mikroemulsionen, die bei Zugabe zum Badewasser klar löslich oder eine milchige Emulsion bilden. Je nach Wahl des Emulgatorsystems kann es hierbei zu einer starken Schaumbildung kommen, die in vielen Fällen erwünscht ist.

Die Emulgatorkonzentration, bezogen auf die Ölphase, liegt gewöhnlich zwischen 1 und 100 %, vorzugsweise zwischen 2 und 60 %. Das Wasser-Öl-Verhältnis liegt gewöhnlich zwischen 100 und 0.2, vorzugsweise zwischen 50 und 0.3.

Die folgenden Beispiele sollen die Erfindung erläutern.

Beispiel 1

2.5 g $C_{12}C_{14}$-Alkylpolyglucosid, Glycosidierungsgrad 1.3 wurde in 10 g Orangenöl (Orangenterpen: Dragoco) bei Raumtemperatur unter geringem Rühren gelöst. Sodann wird die Lösung in 87.5 g Wasser unter geringem Rühren eingebracht. Es bildet sich sofort eine feinteilige Emulsion, die auch nach Standzeiten von 3 Wochen unverändert ist.

Beispiel 2

12.5 g $C_{12}C_{14}$-APG, Glycosidierungsgrad 1.3, wurden in 50 g Orangenöl gelöst und die Lösung wie in Beispiel 1 mit 37.5 g Wasser vereinigt. Es bildet sich eine opaleszierende, sehr feinteilige Emulsion, die sich auch bei erhöhter Temperatur (50 °C) in 1 Woche nicht verändert.

Beispiel 3

2.5 g $C_{10}C_{12}$-APG, Glycosidierungsgrad 1.2, wurden in 10 g Anisöl (Dragoco) unter geringem Rühren fein verteilt. Sodann wird die leicht trübe Lösung in 87.5 g Wasser eingebracht. Es bildet sich sofort eine feinteilige, blau-weiße Emulsion, die auch nach längerer Standzeit stabil ist.

Beispiel 4

45 g $C_{10}C_{12}$-APG, Glycosidierungsgrad 1.7, wurden in 40 g alkoholischem Rosmarinauszug (Kneipp) gelöst, es wurde 1 g Softigen(R)767 (Rückfettmittel) hinzugegeben und mit 14 g Wasser vereinigt. Es entsteht eine klare Flüssigkeit mit einer Viskosität von 36 mPa•s und einem Klarpunkt von 4 °C.

Beispiel 5

2.4 g $C_{12}C_{14}$-APG, Glycosidierungsgrad 1.3 und 0.1 g $C_{12}C_{14}$-Fettalkoholsulfat werden in 10 g Eukalyptusöl (Dragoco) unter geringem Rühren verteilt. Sodann wird die trübe Lösung mit 87.5 g Wasser vereinigt. Es entsteht eine opaleszierende, sehr feinteilige Emulsion, die auch nach längerer Standzeit (3 Wochen) bei 40 °C unverändert war.

Beispiel 6 (Vergleichsbeispiel)

2.5 g $C_{12}C_{14}$-APG, Glycosidierungsgrad 1.3 werden in 10 g Eukalyptusöl verteilt. Bringt man sodann die trübe Lösung in Wasser, kommt es sofort zur Phasentrennung. Das gleiche Ergebnis wird erhalten, wenn statt Alkylpolyglycosid $C_{12}C_{14}$-Fettalkoholsulfat als einziger Emulgator eingesetzt wird.

Beispiel 7

2.4 g $C_{12}C_{14}$-APG, Glycosidierungsgrad 1.3 und 0.1 g $C_{12}C_{18}$-Fettalkoholsulfat werden in 10 g Fichtennadelöl (Dragoco) gelöst. Sodann wird unter geringem Rühren die leicht trübe Lösung mit 87.5 g Wasser vereinigt. Es entsteht eine opaleszierende, extrem feinteilige Emulsion, die auch nach Lagerzeiten von 1 Monat bei 50 °C keinerlei Veränderungen anzeigt. Bei Verwendung der beiden Tenside jeweils allein als Emulgator jedoch tritt sofortige Phasentrennung auf.

Beispiel 8

1.25 g $C_{10}C_{12}$-Alkylpolyglucosid mit einem Glycosidierungsgrad von 1.2 und 1.25 g IMWITOR(R)370 (Zitronensäureester von Mono- und Digliceriden von Speisefettsäuren) werden unter Rühren in 87.5 g Wasser gelöst. Beim Einbringen von 10 g Fichtennadelöl entsteht eine lagerstabile, sehr feinteilige, opaleszierende Emulsion.

Wird IMWITOR(R)370 allein als Emulgator eingesetzt, so tritt sofort Trennung auf.

Beispiel 9

2.35 g $C_{12}C_{14}$-Alkylpolyglycosid mit einem

Glycosidierungsgrad von 1.3 und 0.15 g $C_{12}C_{14}$-Alkoholsulfat wurden unter geringem Rühren in einer Mischung aus 4 g Orangenöl und 6 g Isopropylmyristat verteilt. Beim Eindringen der leicht trüben Lösung in 87.5 g Wasser entsteht eine bläulich-weiße feinteilige Emulsion.

Beispiel 10

2.35 g $C_{12}C_{14}$-Alkylpolyglycosid mit einem Glycosidierungsgrad von 1.3 und 0.15 g $C_{12}C_{14}$-Alkoholsulfat wurden unter Rühren in einer Mischung aus 5 g Eukalyptusöl und 5 g Isopropylmyristat gelöst. Beim Einbringen der leicht trüben Lösung in 87.5 g Wasser entsteht eine weiße, lagerstabile Emulsion.

**Patentansprüche**

1. Emulgatorsysteme zur Herstellung von in der Kosmetik oder Medizin verwendbaren Öl-in-Wasser-Emulsionen etherischer Öle allein oder im Gemisch mit einer Fettphase,
   dadurch gekennzeichnet,
   daß sie Alkylpolyglycoside der Formel (I)

   R - O - $Z_n$     (I)

   enthalten, wobei R für einen linearen oder verzweigten, gesättigten oder ungesättigten Alkylrest mit 8 - 16 C-Atomen, Z für einen Oligoglycosidrest stehen und n im Mittel 1 - 5 bedeutet.

2. Emulgatorsysteme nach Anspruch 1,
   dadurch gekennzeichnet,
   daß in Formel (I)
   R für einen linearen, gesättigten Alkylrest mit 10 bis 14 C-Atomen, Z für einen Oligoglycosidrest stehen, bei dem n im Mittel 1.1 bis 2.0 bedeutet.

3. Emulgatorsysteme nach den Ansprüchen 1 bis 2,
   dadurch gekennzeichnet,
   daß neben Verbindungen der Formel I noch Coemulgatoren enthalten sind.

4. Emulgatorsysteme nach den Ansprüchen 1 bis 3,
   dadurch gekennzeichnet,
   daß im Emulgatorsystem Verbindungen der Formel (I) in Konzentrationen von 80 bis 100 % und Coemulgatoren in Konzentrationen von 0 bis 20 % enthalten sind.

5. Emulgatorsysteme nach den Ansprüchen 1 bis 3,
   dadurch gekennzeichnet,
   daß im Emulgatorsystem Verbindungen der Formel (I) in Konzentrationen von 85 bis 100 % und Coemulgatoren in Konzentrationen von 0 bis 15 % enthalten sind.